# EUROPÄISCHE PATENTSCHRIFT

(11) **EP 3 044 585 B1**
(45) Veröffentlichungstag und Bekanntmachung des Hinweises auf die Patenterteilung: **16.08.2017**
(21) Anmeldenummer: 14793007.7
(22) Anmeldetag: 11.09.2014
(51) Int. Cl.: G01N 33/28, F16N 39/00, F16N 29/00

(54) **VERFAHREN UND EINRICHTUNG ZUR ANALYSE VON ÖLEN UND TECHNISCHEN BETRIEBSFLÜSSIGKEITEN UND ZUR QUALIFIZIERTEN BEWERTUNG DER BETRIEBSZUSTÄNDE VON AGGREGATEN**
METHOD AND DEVICE FOR THE ANALYSIS OF OILS AND TECHNICAL SERVICE FLUIDS AND FOR THE QUALIFIED EVALUATION OF THE OPERATING STATES OF MACHINES
PROCÉDÉ ET SYSTÈME D'ANALYSE D'HUILES ET DE FLUIDES TECHNIQUES ET D'ÉVALUATION QUALIFIÉE DES ÉTATS OPÉRATIONNELS DE MACHINES

(30) Priorität: 12.09.2013 DE 102013110011
(43) Veröffentlichungstag der Anmeldung: 20.07.2016
(73) Patentinhaber: Horstmeyer, Gert, 63633 Birstein (DE)
(72) Erfinder: Horstmeyer, Gert, 63633 Birstein (DE)
(74) Vertreter: Hofmann, Klaus
(86) Internationale Anmeldenummer: PCT/DE2014/100332
(87) Internationale Veröffentlichungsnummer: WO 2015/035983

(56) Entgegenhaltungen:
- EP-A1- 0 635 714
- EP-A1- 0 711 999
- WO-A1-2009/080049
- DE-A1-102005 058 595
- DE-C- 941 520
- US-B1- 6 598 464
- Gerardo Trujillo: "The Blotter Spot Method - Sample Preparation and Test Procedure: A Quick Guide", Practicing Oil Analysis, 1. Juli 2003 (2003-07-01), XP055161107, Gefunden im Internet: URL:http://www.machinerylubrication.com/Re ad/499/blotter-spot-method [gefunden am 2015-01-09]

## Beschreibung

Die Erfindung betrifft ein Verfahren und eine Einrichtung zur Analyse von Ölen und technischen Betriebsflüssigkeiten und zur qualifizierten Bewertung der Betriebszustände von Aggregaten, bei welchem man einen Tropfen der zu untersuchenden Testflüssigkeit auf ein Testmedium aufträgt, in das Testmedium eindringen lässt und nach einer vorgewählten Zeit derart auswertet, indem ein sich ergebendes Bild optisch mit den Daten von mehreren Referenzbildern nach mehreren Testkriterien verglichen wird.

Mit dem Verfahren und der zugehörigen Einrichtung können verschiedene Testflüssigkeiten hinsichtlich unterschiedlicher Merkmale, Inhalte sowie Kontamination untersucht werden, beispielsweise Motorenöle, Hydrauliköle, Getriebeöle, Bremsflüssigkeiten, Industrieöle sowie Koch-, Back- und Lebensmittelöle.

Die Erfindung dient der Analyse von möglichen Beschädigungen durch normalen oder ungewöhnlichen Verschleiß sowie sich anbahnender Schäden in Aggregaten der Automotiv-Industrie, wie beispielsweise Motoren, Bremsen, Servolenkungen oder Getrieben sowie in Anlagen, Getrieben, hydraulischen Mechaniken und Steuerungen von Industrieanlagen mittels der Analyse der entsprechenden Öle oder Betriebsflüssigkeiten. Außerdem dient sie der Analyse der Qualität von Ölen und Betriebsflüssigkeiten in der Industrie, beispielsweise der Lebensmittelindustrie, speziell in Küchen.

In DE 941 520 B wird eine Vorrichtung zum Prüfen von Schmieröl oder ähnlichen Stoffen auf eine darin enthaltene Menge fremder oder verfärbender Bestandteile mit Hilfe eines Saugfleckes auf einem saugfähigen Papier beschrieben. Gemäß der Erfindung wird dieses Ziel dadurch erreicht, dass der größere Teil eines Papierblattes an seiner Oberfläche, z. B. durch einen Farbüberzug, verhältnismäßig nicht saugend oder sogar abweisend für den zu prüfenden Stoff bzw. das Öl ausgebildet ist und in diesem Teil eine Mehrzahl von verhältnismäßig saugfähigen Stellen ausgespart ist, die im Innern Markierungen in der Vergleichsfarbe aufweisen. Der Farbvergleich zwischen je einer saugfähigen an je einer nicht saugfähigen Stelle angebrachten Probe und der dazugehörigen Markierung für unterschiedliche Proben soll dann sehr leicht durchführbar sein.

US 5 313 824 A beschreibt einen Testkit und ein manuelles Verfahren für die Analyse von Schmieröl, bei dem eine Probe für eine bestimmte Zeit auf einem Testmedium aufgebracht wird und einem visuellen Vergleich mit einer Probe unterzogen werden kann.

In DE 10 2005 058 595 A1 und EP 1 825 256 B1 werden ein Testmedium sowie ein Verfahren zur Schnellanalyse von Motorölen in Verbrennungsmotoren beschrieben. Dabei wird ein Tropfen eines zu untersuchenden Öles auf ein spezielles Testmedium aufgetragen und in das Testmedium eindringen gelassen. Nach einer vorgegebenen Zeit erfolgt eine manuelle Auswertung durch eine Person derart, dass das sich ergebende Bild optisch mit mehreren Referenzbildern verglichen wird. In US6598464 B1 werden mehrere Testkriterien für Öle an Hand optischer Messungen bewertet, wobei der Test voll automatisiert erfolgen kann. Mehrere Ölproben werden in Vertiefungen eines Testbehälters eingebracht und einzeln jeweils an Hand der vorgegebenen Kriterien untersucht. Bei der bisherigen optischen Auswertung durch das Auge eines Betrachters können die Ergebnisse der Tests unterschiedlich individuell bewertet werden. Auch sehen menschliche Augen sehr unterschiedlich und nehmen Farbgebungen unterschiedlich wahr. Dieses stellt eine gewisse Unsicherheit dar. Auch werden nicht alle Bestandteile vom Auge erfasst. Bestimmte Bestandteile in den zu untersuchenden Ölen können nicht durch das Auge erkannt werden. Das Verfahren der optischen Auswertung durch das Auge ist lediglich ein Grobraster. Die Bewertung durch den Anwender unterliegt einer Reihe von subjektiven Einflüssen und ist nur mit einem größeren Übungsaufwand erlernbar. Hier spielt auch die menschliche Trägheit eine Rolle, die sich oft kritisch gegenüber Neuigkeiten äußert. Die bisherigen Verfahren sind erklärungsbedürftig und schulungsintensiv. In Grenzfällen ist die Bewertung der Ergebnisse nicht sicher genug.

Die US 5 091 652 A bezieht sich auf einen Fluoreszenzscanner und insbesondere auf einen durch einen Laser zur Fluoreszenz anregenden Geles abtastenden Scanner mit einem konfokalen Mikroskoperfassungssystem.

DE 10 2005 015 826 A1 beschreibt ein Verfahren und ein System zur optischen Inspektion von Kontaktflächen (Kontaktpads) an Halbleiter-Bauelementen mit unterschiedlichem Erscheinungsbild. Die Position von Kontakt-Nadelspitzen auf einem Kontaktpad kann bestimmt werden, indem von der Oberfläche des Kontaktpads vor dem Kontaktieren des Kontaktpads ein erstes Bild aufgenommen wird, nach dem Kontaktieren des Kontaktpads durch ein Kontaktelement ein zweites Bild aufgenommen wird, aus der Differenzbildung zwischen dem ersten und dem zweiten Bild ein drittes Bild erzeugt wird, das analysiert wird, um festzustellen, an welcher Stelle die Kontakt-Nadel einen Abdruck auf dem Kontaktpad hinterlassen hat.

Beide vorgenannten Lösungen sind nicht geeignet, Öle und andere technische Betriebsflüssigkeiten zu analysieren und Rückschlüsse auf Aggregate zu ermöglichen, aus denen sie entnommen wurden.

In EP 0 571 295 A1 wird ein Gerät zur Bemessung eines Schmiermittels, insbesondere für einen Kraftfahrzeugmotor, beschrieben, bei dem eine Frontlichtaufnahme erstellt und hinsichtlich bestimmter Schmiermitteleigenschaften automatisch ausgewertet wird.

Ein in DE 196 37 234 A1 beschriebenes Verfahren dient zur Online-Überprüfung der Farbreinheit von Oberflächen mit einer aus einer oder mehreren Videokameras und einem Computer bestehenden Einrichtung, insbesondere zum Zwecke der Qualitätskontrolle.

In US 4 781 892 A wird eine Vorrichtung und ein Verfahren zur Feststellung der Verschmutzungsanfälligkeit von flüssigen Kohlenwasserstoffen beschrieben, bei denen eine Frontaufnahme in Form von reflektiertem Licht und eine Rücklichtaufnahme in Form von Transmittiertem Licht erstellt werden. Eine Auswertung erfolgt über die Lichtintensität.

Aufgabe der Erfindung ist es, ein Verfahren und eine Einrichtung zur Analyse von Ölen und technischen Betriebsflüssigkeiten vorzuschlagen, bei denen die zu untersuchenden Testflüssigkeiten Aufschluss über deren Zustand selbst und Aussagen und Bewertungen über den mechanisch-technischen Zustand der mit den Testflüssigkeiten verbundenen Komponenten und Aggregaten vorgenommen werden können. Die Erfindung soll eine schnelle und zuverlässige Erfassungs-, Auswerte- und Bewertungsmöglichkeit bieten. Der Einfluss von subjektiven Bewertungseinflüssen soll reduziert oder ganz ausgeschlossen werden.

Die Erfindung löst die Aufgabe durch die in den Ansprüchen 1 und 12 angegebenen Merkmale. Vorteilhafte Weiterbildungen der Erfindung sind in den Unteransprüchen angegeben und werden nachstehend zusammen mit den beschriebenen Ausführungen der Erfindung, einschließlich der Zeichnungen, näher erläutert.

Das Verfahren zur Analyse von Ölen und technischen Betriebsflüssigkeiten und zur qualifizierten Bewertung der Betriebszustände von Aggregaten geht davon aus, dass ein Tropfen der zu untersuchenden Testflüssigkeit auf ein geeignetes Testmedium aufgetragen wird, dass dieser Tropfen in das Testmedium eindringt und sich nach einer bestimmten Zeit ein auswertbares Bild ergibt. Dieses Testbild wird optisch mit den Daten von mehreren Referenzbildern nach bestimmten Testkriterien verglichen.

Als vorbereitende Maßnahme werden Reihen von Referenzbildern für verschiedene Testkriterien aufgenommen und als Daten abgespeichert. Diese Referenzbilder werden zu bestimmten Eigenschaften der zu untersuchender Flüssigkeiten und Betriebszuständen von Aggregaten zugeordnet, sodass jedem Referenzbild ein bestimmter Zustand zugeordnet wird.

Zur eigentlichen Analyse wird das sich auf dem Testmedium bildende Testbild des Tropfens mit einem Bildaufnahmegerät mindestens in einer Frontaufnahme und einer Rücklichtaufnahme aufgenommen und abgespeichert. In einer besonderen Ausführung kann die Rücklichtaufnahme eine UV-Lichtaufnahme sein. In einer bevorzugten Ausführung kann zusätzlich zur Rücklichtaufnahme mit weißem und/oder farbigem Licht eine UV-Rücklichtaufnahme erfolgen. Anschließend erfolgt ein rechnergestützter Vergleich der Daten der Testbilder mit den Daten der Referenzbilder, indem die Daten eines oder mehrerer Bilder aus der Front-, der Rücklicht- und ggf. der UV-Rücklichtaufnahme zu den Referenzbildern für jeweils ein Testkriterium, welches die größte Übereinstimmung aufweist, zugeordnet wird. Anschließend erfolgen eine Anzeige und/oder eine Protokollierung der Eigenschaften der Testflüssigkeit und/oder des Betriebszustandes des Aggregates, die den zugeordneten Daten der Referenzbilder entsprechen.

Da die einzelnen zu untersuchenden Testflüssigkeiten sehr unterschiedliche Eigenschaften aufweisen und auch der Zustand der unterschiedlichen Aggregate, aus denen die Testflüssigkeiten entnommen wurden, wie beispielsweise Bremse, Servolenkung, Getriebe oder mittels Hydraulik gesteuerte Anlagen oder Komponenten wie Servolenkungen, durch sehr unterschiedliche Kriterien beeinflusst sein kann, sollte für jedes Testmedium und für jedes Aggregat eine eigene Reihe an Referenzbildern erstellt und der Analyse zugrunde gelegt werden. Sind diese Referenzbilder einmal erstellt, können diese immer wieder der Analyse und Auswertung zugrunde gelegt werden. So wird es auch möglich sein, dass neue Testflüssigkeiten oder Aggregate sehr einfach und schnell in das Analysesystem integriert werden können.

Der rechnergestützte Vergleich der Daten der Testbilder mit den Daten der Referenzbilder erfolgt vorzugsweise mittels einer Software, die geeignet ist, die Form und/oder die Farbspektren der Daten der Testbilder und die Daten der Referenzbilder zu unterscheiden und die Daten der Bilder aus der Front-, der Rücklicht- und ggf. der UV-Rücklichtaufnahme zu je einem Referenzbild für jeweils ein Testkriterium zuzuordnen.

Die Aufnahme und Speicherung der Daten der Referenzbilder kann auf einem internen Rechner oder auf einem externen Server erfolgen. Bei einer externen Ablage kann der rechnergestützte Vergleich auch auf dem externen Server erfolgen, wobei vom Aufnahmegerät der Testbilder dann eine Verbindung bestehen muss, beispielsweise eine USB- oder eine Online-Verbindung.

Für die Frontaufnahme und/oder die Rücklichtaufnahme können unterschiedlich farbige Lichtquellen eingesetzt werden, die die Auswertung der sich ergebenden Testbilder bei bestimmten Testkriterien verbessern können. Auch das Bildaufnahmegerät selbst kann fotografische Filter enthalten, beispielsweise spezielle Farb- oder Graufilter.

Zu den eigentlichen Testaufnahmen kann durch das Bildaufnahmegerät eine Aufnahme zur Identifizierung und Dokumentation für eine Zuordnung der Testflüssigkeit zu einer Probennahme, beispielsweise Art der Testflüssigkeit, Zeitpunkt der Aufnahme und/oder einem Objekt, beispielsweise einem bestimmten Fahrzeug, und/oder einer Person, beispielsweise eines Auftraggebers, erstellt werden. Dazu kann beispielsweise auch eine Frontaufnahme genutzt werden.

Als Testmedium können verschiedene geeignete beschichtete oder unbeschichtete Trägermaterialien wie Filter- oder Laborpapier, Kunststofffolien, Aluminiumfolien verwendet werden. In einer bevorzugten Ausführung wird ein Testmedium verwendet, welches ein Flächengewicht von 50,0 g/m² bis 200,0 g/m² aufweist und welches aus, bezogen auf das Gesamtgewicht des Testmediums, 70,0 Gew.-% bis 98,0 Gew.-% Baumwollpulp, 0,0 Gew.-% bis 25,0 Gew.-% Cellulose und 0,5 Gew.-% bis 30,0 Gew.-% Kieselsäure und/oder mindestens eines Silikates besteht.

Das beschriebene Verfahren ist geeignet, die Testflüssigkeiten hinsichtlich verschiedener Testkriterien zu untersuchen. Es sind mehrere Varianten möglich, um zu einem auswertbaren Testergebnis zu gelangen. So kann jede einzelne Aufnahme aus der Front- der Rücklicht- und ggf. der UV-Rücklichtaufnahme mit jeweils einer Reihe von Referenzaufnahmen für jeweils nur ein Testkriterium verglichen werden oder es wird eine Aufnahme mit mehreren Reihen von Referenzaufnahmen hinsichtlich gleich mehrerer Testkriterien untersucht und ausgewertet.

Testkriterien können beispielsweise bei Motoren der Rußgehalt, Metallanteile oder Sedimente, der Zustand des Motoröles selbst, seine Alterung, ein Additivabbau oder der Gehalt an Kondens- oder Kühlwasser bzw. an Treibstoff sein; bei Bremsen der Abrieb, ein Schmutzeintrag, der Wassergehalt oder der Additivabbau (Versumpfung); bei Servolenkungen der Abrieb, der Schmutzeintrag oder der Additivabbau und bei Getrieben ein Metallabrieb oder ein Wassereintrag durch Kühlwasser, beispielsweise bei Automatikgetrieben.

Zur Analyse des Gesundheitszustands von Aggregaten kann Testflüssigkeit hinsichtlich enthaltener Verunreinigungen durch Ruß, Staub, Metallabrieb untersucht werden, indem ein in der Mitte des Testbildes in einem Bereich a entstehender Rußfleck mit den Daten der Referenzbilder verglichen und der Gehalt an Verunreinigungen ermittelt und bewertet wird.

Zur Analyse des Gesundheitszustands von Aggregaten kann die Testflüssigkeit hinsichtlich des Zustandes der Testflüssigkeit selbst untersucht werden, indem ein in einem Bereich b des Testbildes entstehender Kreisring bezüglich seiner Farbe mit den Daten der Referenzbilder verglichen und die Qualität der Testflüssigkeit in Relation zu seiner Alterung, beispielsweise der Öllaufleistung, ermittelt und bewertet wird.

Eine weitere Möglichkeit besteht darin, dass zur Analyse des Gesundheitszustandes von Aggregaten Testflüssigkeit hinsichtlich des Gehaltes an Wasser und/oder im Wasser vorhandene Kühlflüssigkeit, beispielsweise Glykol, untersucht werden kann, indem eine in einem Bereich c des Testbildes entstehende gezackte Randzone bezüglich seiner Form mit den Daten der Referenzbilder verglichen und der Wassergehalt der Testflüssigkeit in Relation zum Zustand des Aggregates ermittelt und bewertet wird.

Zur Analyse des Gesundheitszustands von Aggregaten kann die Testflüssigkeit hinsichtlich des Gehaltes an Kraftstoff untersucht werden, indem ein am äußeren Ring des Testbildes in einem Bereich d entstehender Treibstoffkreis bezüglich seiner Form und Farbe mit den Daten der Referenzbilder verglichen und der Treibstoffgehalt der Testflüssigkeit in Relation zum Zustand des Aggregates ermittelt und bewertet wird.

Die erfindungsgemäße Einrichtung zur Analyse von Ölen und technischen Betriebsflüssigkeiten und zur qualifizierten Bewertung der Betriebszustände von Aggregaten umfasst eine Auflage oder eine Halterung für ein flächenhaftes Testmedium, beispielsweise eine Trägerplatte. Auf dem Testmedium ist ein Tropfen der zu untersuchenden Testflüssigkeit auftragbar und ein Testbild erstellbar. Die Auflage sollte klar oder zumindest lichtdurchlässig sein, um Rücklichtaufnahmen zu ermöglichen und nicht zu verfälschen.

Zur Aufnahme der Testbilder besitzt die Einrichtung mindestens ein Bildaufnahmegerät für eine Frontaufnahme und eine Rücklichtaufnahme und mindestens je eine Lichtquelle zur Bilderfassung für eine Frontaufnahme und einer Rücklichtaufnahme. Das Bildaufnahmegerät kann ein Fotoapparat oder ein optisch-digitaler Scanner beispielsweise ein Laserscanner sein. Es können jeweils ein oder mehrere Bildaufnahmegeräte vorhanden sein oder es können mehrere unterschiedliche Bildaufnahmegräte miteinander kombiniert werden. In einer besonderen Ausführung kann die Rücklichtaufnahme eine UV-Rücklichtaufnahme sein. In einer bevorzugten Ausführung kann zu einer Rücklichtaufnahme mit vorzugsweise Normallicht eine zusätzliche UV-Rücklichtaufnahme erfolgen. Für beide Varianten sind entsprechende Lichtquellen zur Bilderfassung für die UV-Rücklichtaufnahme vorzusehen.

Zur Einrichtung gehören weiterhin Mittel zur Speicherung des sich auf dem Testmedium bildenden Testbildes sowie Mittel zur Speicherung der Daten mehrerer Reihen von Referenzbildern. Die Mittel zur Speicherung des sich auf dem Testmedium bildenden Testbildes sind insbesondere ein interner Datenspeicher, der an einen Rechner angebunden ist. Auch im Bildaufnahmegerät können sich die üblichen Bildspeicher befinden, zumindest für eine Zwischenspeicherung. Die Mittel zur Speicherung der Daten mehrerer Reihen von Referenzbildern befinden sich als Datenspeicher entweder in einem internen Rechner oder einen externen Server mit einer üblichen Ausstattung wie Prozessoren, Datenspeichern, Stromversorgern und zugehörigen Treibern und Software.

Weiterhin gehören zur Einrichtung Mittel, insbesondere eine Software, die geeignet ist, die Daten der Testbilder bezüglich ihrer Form und Farbspektren mit den Daten der Referenzbilder zu vergleichen und die Daten je eines Bildes oder mehrere Bilder aus der Front-, der Rücklicht- und ggf. der UV-Rücklichtaufnahme zu je einem Referenzbild oder Referenzbildern für jeweils ein Testkriterium zuzuordnen, welches die größte Übereinstimmung aufweist. Diese Software kann sich entweder auf einem internen Rechner oder einem externen Server befinden, je nachdem wo die Auswertung erfolgen soll.

Schließlich besitzt die Einrichtung eine Anzeige- und/oder eine Protokolliereinheit zur Darstellung und/oder Ausgabe der Analyse- und/oder Bewertungsergebnisse.

Die Auflage, die Bildaufnahmegeräte, die Lichtquellen sowie ggf. die Anzeige- und/oder die Protokolliereinheit befinden sich vorzugsweise innerhalb eines Gehäuses, welches vor äußeren Einflüssen geschützt sein sollte. Die Auflage bzw. die Halterung für das Testmedium kann eine zusätzliche Arretierung besitzen, die das flächenhafte Testmedium immer an einer bestimmten Stelle platziert, damit vergleichbare Aufnahmen erstellt werden können. Außerdem kann eine Schutzvorrichtung zum Schutz vor Verschmutzung der Aufnahmegeräte, der Lichtquellen oder des Testmediums vorgesehen sein, z.B. zusätzliche Glasscheiben.

Eine besondere Ausführung der Erfindung betrifft eine Einrichtung, bei der das eigentliche Testaufnahmegerät eine Schnittstelle zu einem Netzwerk und/oder zum Internet besitzt und sich die Mittel zur Speicherung der Daten mehrerer Reihen von Referenzbildern sowie die Software für den Vergleich und die Zuordnung der Daten der Testbilder mit den Daten der Referenzbilder auf einem externen Server befinden. Die Einrichtung ist dann über eine Online-Verbindung als Client-Server-Gerät betreibbar.

Es ist weiterhin möglich, dass das Testaufnahmegerät nur der Aufnahme der Daten der Testbilder und ggf. der Referenzbilder dient und die Speicherung auf einem unmittelbar angeschlossenen Rechner erfolgt, von dem aus dann ggf. eine Verbindung zu einem externen Server erfolgt, auf dem die Auswertung stattfindet. Die Anzeige, Protokollierung oder Ausgabe der Testergebnisse kann dann entweder am Testaufnahmegerät selbst oder am unmittelbar angeschlossenen Rechner erfolgen.

Die Einrichtung kann somit als ein stationäres Gerät, ein mobiles Endgerät mit einer eigenen Stromversorgung oder eine Kombination aus beiden sein.

Die Vorteile der vorliegenden Erfindung liegen darin, dass die optisch-elektronische Methode mehr Sicherheit in der Erfassung und Auswertung bietet. Unterschiedliche Interpretationen, wie mit dem menschlichen Auge, sind weitestgehend ausgeschlossen. Das Verfahren ist schneller und präziser, da die Bestandteile in den Ölen genauer ermittelt werden können und hierdurch wesentlich genauere Bewertungen vorliegen werden. Der Blick in die Komponenten und Aggregate wird durch dieses Verfahren viel genauer und die Aussagen fundierter. Das führt zu besseren Ergebnissen in diesen Testverfahren. Die Erfindung bietet ebenfalls weitere Vorteile bei der Darstellung, Übermittlung und Archivierung der Testergebnisse.

Die Erfindung ermöglicht einen indirekten Blick in den Zustand von Motoren und Aggregaten, ohne diese öffnen oder zerlegen zu müssen.

Nachfolgend wird die Erfindung an einem Ausführungsbeispiel näher erläutert. Es zeigen:
- Fig. 1: Einrichtung mit Bilderfassung durch ein Kamerasystem
- Fig. 2: Einrichtung mit Scanner und klappbarem Deckel
- Fig. 3: Darstellung von vier Testkriterien und von vier Reihen mit Referenzbildern

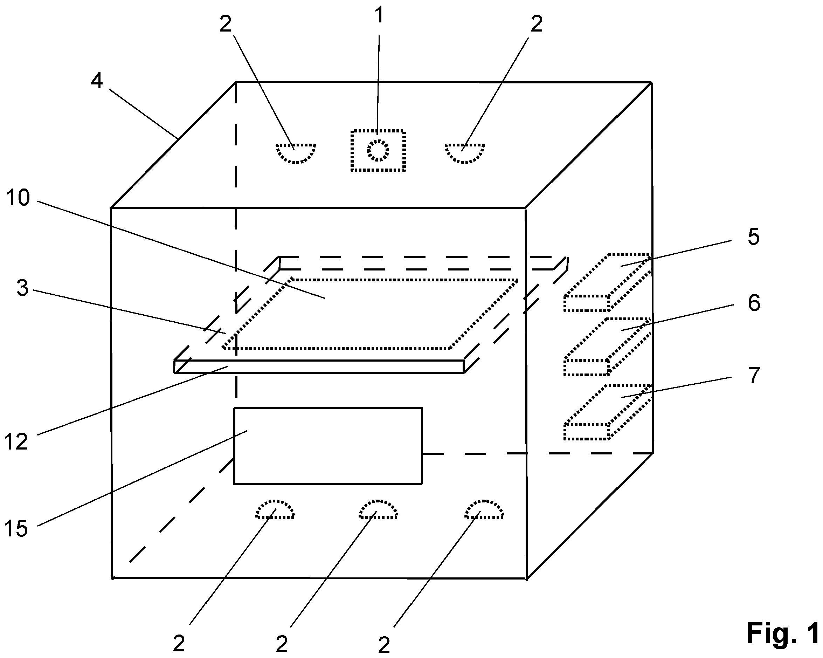

Eine beispielhafte Einrichtung ist in Fig. 1 dargestellt. Sie zeigt eine Einrichtung zur Analyse von Ölen und technischen Betriebsflüssigkeiten und zur qualifizierten Bewertung der Betriebszustände von Aggregaten mit einer optisch-elektronischen Bilderfassung durch ein Kamerasystem.

Ein abgeschlossenes Gehäuse 4 besitzt einen Aufnahmeschlitz 12 zur Einführung eines Blattes eines Testmediums 10, auf welches ein Tropfen der zu untersuchenden Testflüssigkeit auftragbar und ein Testbild erstellbar ist. Hinter dem Aufnahmeschlitz 12 befindet sich eine Auflage für das Testmedium 10, die als gläserne Trägerplatte 3 ausgebildet ist. Oberhalb der Trägerplatte 3 mit dem Testmedium 10 ist eine Kamera 1 befestigt, die geeignet ist, mehrere Front-, Gegenlicht- und UV-Rücklichtaufnahmen zu erstellen und diese in einem Rechner 5 abzulegen. Neben der Kamera 1 sind zwei Lichtquellen 2 für Frontaufnahmen angebracht. Unterhalb der Trägerplatte 3 sind drei weitere Lichtquellen 2 vorgesehen, wobei die linke Lichtquelle 2 weißes Licht, die mittlere Lichtquelle 2 UV-Licht und die rechte Lichtquelle 2 gelbes Licht erzeugt. Das weiße und das gelbe Licht sind für Rücklichtaufnahmen und das UV-Licht für UV-Rücklichtaufnahmen vorgesehen.

Im vorliegenden Beispiel werden die Daten mehrerer Reihen von Referenzbildern intern im gleichen Speicher des Rechners 5 abgelegt, wo auch die Testbilder gespeichert werden. Der Rechner 5 enthält eine Software, die geeignet ist, die Daten der Testbilder bezüglich ihrer Form und Farbspektren mit den Daten der Referenzbilder zu vergleichen und die Daten je eines Bildes oder mehrerer Bilder aus der Front-, der Rücklicht- und der UV-Rücklichtaufnahme zu je einem Referenzbild oder Referenzbildern für jeweils ein Testkriterium zuzuordnen, welches die größte Übereinstimmung aufweist. Im Ergebnis dieser Auswertung werden die Analyse- und Bewertungsergebnisse auf einem der vorhandenen Datenspeicher, beispielsweise dem Rechner 16, protokolliert und auf der Anzeigeeinheit 15 angezeigt. Im Rechner 5 befindet sich auch die Software zur Steuerung der Abfolgeschritte zur Erstellung der Testbilder.

Im Gehäuse 4 des Testaufnahmegerätes befinden sich noch ein Energieanschluss 6 sowie eine Schnittstelle 7 für externe Anschlüsse, beispielsweise einen USB-Anschluss für externe Speichermöglichkeiten oder zur Ausgabe der Ergebnisse. Außerdem kann die Schnittstelle 7 eine Netzwerkkarte für einen Internetanschluss enthalten.

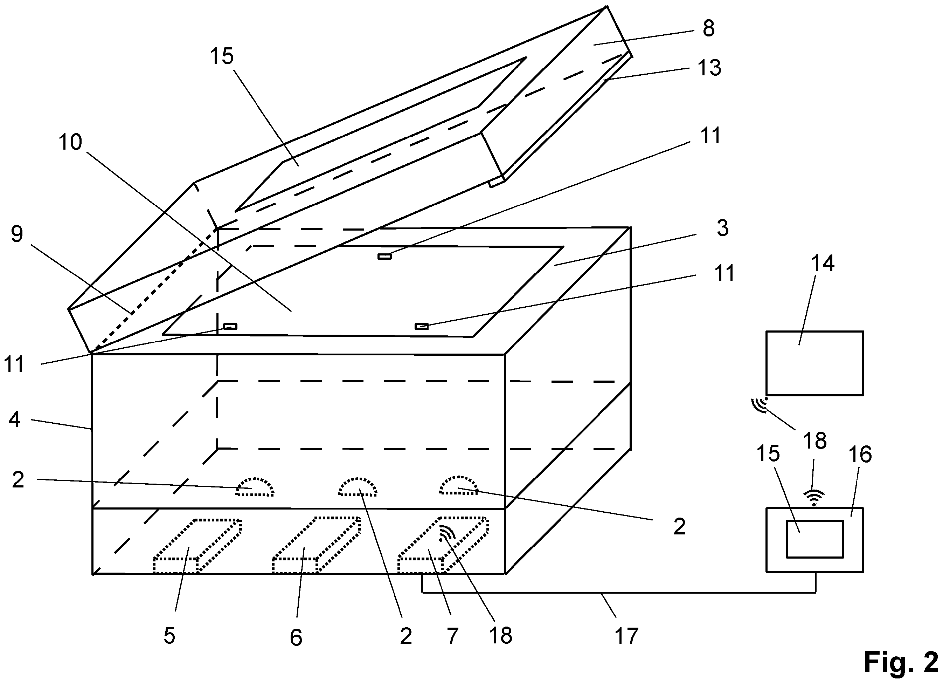

In Fig. 2 ist ein weiteres Beispiel dargestellt. Statt eines Aufnahmeschlitzes erfolgt hier die Aufnahme des Blattes eines Testmediums 10 mit dem Tropfen der zu untersuchenden Testflüssigkeit direkt auf einer gläsernen Trägerplatte 3. Im Testmedium 10 sind 3 Arretierungslöcher ausgespart, die mit den 3 Arretierungen 11 auf der Trägerplatte 3 korrespondieren, sodass das Testmedium 10 genau in diesen Arretierungen 11 fixiert werden kann und bei der Bilderfassung immer vergleichbare Bedingungen vorherrschen.

Bevor die Testbilder mit einer als Bildaufnahmegerät dienenden Scannereinheit 13 erstellt werden, wird der um ein Scharnier 9 klappbare Deckel 8 geschlossen. Je nachdem, ob der Scanner unter- oder oberhalb des Testmediums angeordnet ist, können entweder Front- oder Rücklichtaufnahmen erfolgen. Zusätzlich befinden sich im Gehäuse noch weitere Lichtquellen 2, beispielsweise für UV- oder Farbaufnahmen.

Zur Aufnahme und Digitalisierung der Test- oder auch der Referenzbilder können angepasste Flachbettscanner mit fahrbarem Schlitten verwendet werden. Bei Flachbettscannern werden die zu scannenden Testmedien auf ein Vorlagenglas gelegt, mit einer Fluoreszenz- oder Halogenlichtquelle ausgeleuchtet und durch einen Deckel 8 gegen die Raumhelligkeit geschützt. Aufsichtsvorlagen werden dabei von unten und Durchsichtsvorlagen von einer Durchlichteinheit von oben beleuchtet. Die Lichtquelle, die gesamte Optik und die CCD-Sensoren sind auf einem Schlitten montiert, der in Längsrichtung stetig unter der Vorlage weggezogen wird. Das reflektierte Licht wird mit Hilfe zweier Spiegel und einer synchron fokussierenden Linseneinheit auf eine gerade Reihe von mehreren Tausend CCD-Sensoren gelenkt und in voller Breite in aufeinanderfolgenden Zeilen digitalisiert. Die Durchlichteinheit wird statt der Abdeckung montiert und enthält eine Lampe, die parallel zum Schlitten über die Vorlage bewegt wird und diese dabei von oben ausleuchtet.

Das Aufnahmegerät kann statt mit einem Aufnahmeschlitz 12 oder mit einem klappbaren Deckel 8 auch mit einem abnehmbaren Deckel versehen sein.

Die Verarbeitung und Auswertung erfolgt in ähnlicher Weise wie im Beispiel nach Fig. 1, nur dass die Referenzbilder sowie die Software zur Auswertung nicht innerhalb des Gehäuses des Testaufnahmegerätes angeordnet sind, sondern auf einem externen Server 14, und die Einrichtung über eine Funkverbindung 18 als Client-Server-Gerät betrieben wird.

Vorbereitend werden für verschiedene Testflüssigkeiten unterschiedliche Referenzbilderreihen erstellt. Da beispielsweise Diesel- und Benzinmotoren unterschiedliche Bilder liefern, sind diese auch getrennt auszuwerten. Auch können gasbetriebene und mit Biodiesel betriebene Motoren ausgewertet werden.

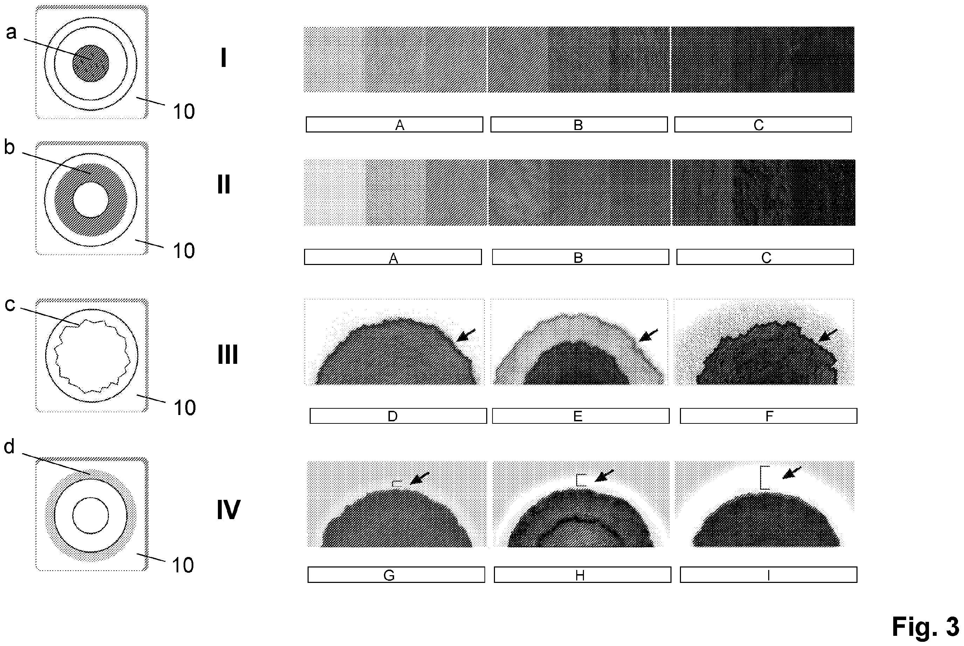

Die Funktionsweise soll nachfolgend anhand eines in Fig. 3 dargestellten Beispieles erläutert werden, bei dem ein Tropfen eines Motoröles eines Dieselmotors analysiert werden soll.

Für das hier beschriebene Beispiel soll das Motorenöl nach 4 verschiedenen Testkriterien I bis IV ausgewertet werden. In Fig. 3 ist in den untereinanderliegenden linken 4 Abbildungen dargestellt, welche Bilder sich durch das Auftröpfeln des Tropfens auf dem Testmedium 10 bilden und welche Bereiche der Bilder nach bestimmten Kriterien ausgewertet werden. Nachfolgend wird die Bedeutung der entstehenden Bilder erläutert.

Testkriterium I - Verrußung und Verunreinigung durch Staub, Metallabrieb usw. Der innere Kreis zeigt in einem Bereich a die Verrußung und Verunreinigung durch Staub, Metallabrieb usw. an. Je nach Verbrennungszustand des Motors und möglicherweise vorhandenem Abrieb oder Staub entsteht ein Rußfleck. Ist das Öl schon sehr viele Kilometer im Motor gelaufen, können sogar die Kreise 2 und 3 vom Ruß überdeckt werden. Ein "gesunder" Motor mit einer Öllaufleistung von 5.000 oder 10.000 km weist die Ringe deutlich auf. Für dieses Testkriterium wird eine Testreihe in 9 unterschiedlichen Testzuständen erstellt und als Referenzbilder für einen späteren rechnergestützten Vergleich auf einem zentralen Server abgelegt. Die Referenzbilderreihe mit den 9 Referenzbildern für das Testkriterium I ist in Bild 3 rechts dargestellt.

### Testkriterium II - Zustand des Motoröls

Der zweite Kreis zeigt in einem Bereich b den Zustand des Motoröls an. Bei Benzinmotoren gilt: je älter das Öl, desto dunkelbrauner wird es, bei Dieselmotoren von Hellgrau nach Tiefschwarz. Rußt ein Motor sehr stark, ist oft kein Unterschied zwischen den Kreisen 1 und 2 zu erkennen. Ist das Öl noch in Ordnung, der Motor rußt aber stark, bildet sich um den braunen (bei Diesel schwarzen) Fleck in der Mitte ein weiterer heller Kreis, der so den Zustand des Motoröls als in Ordnung anzeigt. Für dieses Testkriterium wird eine Testreihe in 9 unterschiedlichen Testzuständen erstellt und als Referenzbilder für einen späteren rechnergestützten Vergleich auf einem zentralen Server abgelegt. Die Referenzbilderreihe mit den 9 Referenzbildern für das Testkriterium II ist in Bild 3 rechts dargestellt.

### Testkriterium III - Wasser im Öl

Die gezackte Randzone c (Pfeil) um den zweiten Kreis zeigt Wasser im Öl. Die Testflüssigkeit beginnt dann sofort nach dem Einziehen des Öls an den Rändern Zacken zu bilden. Wenn viel Wasser im Öl ist, erkennt man dieses bereits nach wenigen Minuten. Bei hohem Wassergehalt können die Ergebnisse von 1,2 und 4 überlagert werden. Es werden zwei Arten von Wasser unterschieden: Kondenswasser und Kühlwasser (mit Glykol). Beide bilden Zacken am Rand. Ist Glykol im Motoröl, erkennt man dies nach etwa 1/2 Stunde an dem äu-βeren, gelben Ring ("Korona") um den Zackenkranz, der stetig größer und deutlicher wird. Für dieses Testkriterium wird eine Testreihe in 3 unterschiedlichen Testzuständen erstellt und als Referenzbilder für einen späteren rechnergestützten Vergleich auf einem zentralen Server abgelegt. Die Referenzbilderreihe mit den 3 Referenzbildern für das Testkriterium III ist in Bild 3 rechts dargestellt.

### Testkriterium IV - Kraftstoff im Öl

Der äußere Ring d bildet den Treibstoffkreis. Befindet sich Kraftstoff im Öl, dann bildet sich ein heller, transparenter Ring außen herum. Je größer der Ring wird, desto mehr Kraftstoff ist im Öl (Pfeil). Ein heller Ring wird sich immer bilden - je größer dieser im Verhältnis zum Tropfen ist, desto mehr Kraftstoff ist vorhanden. Wenn nach einigen Stunden ein Ring erkennbar wird, so ist das als "mittel" einzustufen und in Ordnung. Für dieses Testkriterium wird eine Testreihe in 3 unterschiedlichen Testzuständen erstellt und als Referenzbilder für einen späteren rechnergestützten Vergleich auf einem zentralen Server abgelegt. Die Referenzbilderreihe mit den 3 Referenzbildern für das Testkriterium IV ist in Bild 3 rechts dargestellt.

Nun kann das eigentliche Analyseverfahren erfolgen. Ein Tropfen des zu untersuchenden Motoröles wird auf ein flächenhaftes Testmedium aufgetragen. Das etwa 50 mm x 50 mm große Testmedium besteht beispielhaft aus einem Trägermaterial mit einem Flächengewicht von 140 g/m², bezogen auf das Gesamtgewicht des Testmediums. Es besteht aus 100 Gew.% Baumwollpulp sowie 0 Gew.-% Cellulose. Als Katalysator, Beschleuniger und Reaktionsmittel ist das Testmedium mit speziellen Stoffen beschichtet.

Nach einer Eindringzeit von etwa 0,15 bis zu 45 min (Je nach Belastung des Öls) sowie einer Reifezeit von 1 - 8 Stunden kann das entstandene Bild ausgewertet werden. Dazu wird das Testmedium in den dafür vorgesehenen Schlitz des Testaufnahmegerätes geschoben oder, wie bei Fig. 2, auf die Trägerplatte 3 gelegt und mittels 3 Arretierungen 11 fixiert. Das Testaufnahmegerät wird eingeschaltet und mittels der enthaltenen Kamera 1 und Lichtquellen wird eine Front-, eine Rücklicht- und eine UV-Rücklichtaufnahme erstellt und digital im mittels USB-Verbindung 17 angeschlossenen Rechner 16 abgespeichert.

Der Bediener des Testaufnahmegerätes wird nun aufgefordert, die Auswertung zu starten. Dabei werden die Testbilder über die im Testaufnahmegerät oder im angeschlossenen Rechner 16 enthaltene Schnittstelle 7 per Internet an den externen Server 14 übertragen, auf dem bereits die Referenzbilder abgespeichert wurden.

Auf dem externen Server 14 befindet sich eine Software, die geeignet ist, die Daten der Testbilder bezüglich ihrer Form und Farbspektren mit den Daten der Referenzbilder zu vergleichen und die Daten je eines Bildes oder mehrerer Bilder aus der Front-, der Rücklicht- und der UV-Rücklichtaufnahme zu je einem Referenzbild oder Referenzbildern für jeweils ein Testkriterium zuzuordnen. Das sich ergebende Testbild wird im Server nun mit den Daten von mehreren Referenzbildern nach mehreren Testkriterien verglichen. Die Software ermittelt dabei für jedes der 4 Testkriterien, mit welchem Referenzbild das jeweilige Testbild die größte Übereinstimmung aufweist.

Da jedem Referenzbild oder mehreren Referenzbildern ein bestimmtes Testergebnis zugeordnet wurde, kann nach der Entscheidung, welches der Referenzbilder dem Testbild am nächsten kommt, auch angegeben werden, welche Eigenschaften dem jeweiligen Testbild zuzuordnen sind.

Es gibt verschiedene Möglichkleiten der Auswertung von Testbildern. Grundsätzlich besteht die Möglichkeit, ein Testbild nach mehreren Kriterien auszuwerten. Es hat sich als vorteilhaft erwiesen, wenn mehrere Aufnahmen erstellt werden und für bestimmte Testkriterien auch besonders geeignete Aufnahmen aus der Front-, der Rücklicht- oder der UV-Rücklichtaufnahme ausgewählt werden. Zur Aufnahme und Auswertung des vorgenannten Testkriteriums I und II ist in vielen Fällen bereits eine Frontaufnahme ausreichend. Außerdem wird die Frontaufnahme dazu genutzt, um eine Identifizierung und Zuordnung zum jeweiligen Test zu erstellen und zu dokumentieren. Die Rücklichtaufnahme wird insbesondere zur Identifizierung und Auswertung der Testkriterien 3 und 4 eingesetzt. Durch die Verwendung von farbigem Licht kann die Auswertung in speziellen Farbspektren unterstützt werden. Die UV-Rücklichtaufnahme erfolgt insbesondere zur Spektralfarbenauswertung des inneren Kreises nach dem Testkriterium 1 und des Zustandes des Motorenöls nach dem Testkriterium 2. Als Rücklichtaufnhme ist die UV-Rücklichtaufnahme auch zusätzlich geeignet, vorhandenes Kondens- oder Kühlwasser nach Testkriterium 3 und das Vorhandenseins von Treibstoff nach Testkriterium 4 zu verdeutlichen.

Im vorliegenden Beispiel nach Fig. 3 ist dargestellt, wie vier Testbilder nach jeweils einem Testkriterien untersucht und ausgewertet werden. In der oberen Reihe I der Fig. 3 ist links ein Testmedium 10 mit einem Tropfen Motoröl eines Dieselmotores dargestellt.

Die Testergebnisse in den Referenzbildern nach Fig. 3 besitzen folgende Bedeutung:

| | |
|---|---|
| Reihe I: | Ruß und Verunreinigungen (Verbrennungsrückstände) |
| Bereich A: | Das Prüfergebnis in diesem Bereich bedeutet, dass sich im Motor keine erhöhten Ruß- und/oder Schmutzanteile befinden. |
| Bereich B: | Im Motor ist eine erhöhte Ruß- und Schmutzbildung vorhanden. Es wird empfohlen, in absehbarer Zeit eine Fachwerkstatt aufzusuchen, um eine genauere Diagnose durchführen zu lassen. |
| Bereich C: | Es wird empfohlen, ab Bereich C der Skala dringend eine Fachwerkstatt aufzusuchen. |

Die Ursachen für schlechte Ergebnisse können eine unvollkommene Verbrennung, schlechte Einstellung des Vergasers, falsche Fahrweise, mangelhafte Einspritzung, verstopfter Hauptstromfilter, Fehler am Auspuff oder Turbolader sein. Die Folgen wären ein möglicher Rußaufbau an Ventilen und die Kolben führen zu einem verschlechterten Wärmeaustausch. Dadurch kommt es zu erhöhtem Verschleiß und Kraftstoffverbrauch sowie zu verschlechterten Emissionswerten.

| | |
|---|---|
| Reihe II: | Zustand des Motoröles (Oxidation und Alterung des Motoröles) |
| | |
| Bereich A: | Das getestete Öl befindet sich in einem guten Zustand. |
| Bereich B: | Bei einem solchen Testergebnis zeigt das Öl erste Alterungserscheinungen. Die Laufleistung des Öls ist mit in Betracht zu ziehen. Eine geringe Laufleistung bei starker Ölalterung deutet auf mögliche Schäden hin. |
| Bereich C: | Es wird ein rechtzeitiger Ölwechsel empfohlen. |

Die Ursachen für ein schlechtes Prüfergebnis deuten auf ein zu langes Wechselintervall, eine kurzzeitige Überhitzung, falsches Öl oder ein zu hoher Schwefelgehalt des Kraftstoffs hin. Die Folgen könnten sein, dass verbrauchtes Motoröl zu einem höheren Verschleiß an Kolbenringen und Motorteilen führt. Es kommt zu höheren Verbrauchswerten bei einer sinkenden Motorleistung.

| | |
|---|---|
| Reihe III: | Wasser im Öl (Kondenswasser oder Kühlwasser, Glykol) |
| | |
| Bereich D: | Es befindet sich kein Wasser im Öl. |
| Bereich E: | Im Öl ist ein leicht bis mittel erhöhter Wasseranteil vorhanden. Es wird ein erneuter Test innerhalb der nächsten 1.000 km und bei Verschlechterung ein Besuch bei einer Fachwerkstatt empfohlen. |
| Bereich F: | Es befindet sich zu viel Wasser im Öl. |

Ursachen für zu viel Wasser im Öl könnten sein, dass Wasser durch fehlerhafte Dichtungen, poröse Ölkühlerdichtungen, defekte Zylinderkopfdichtung, Risse im Kühlwasserkreislauf oder korrodierende Lötstellen am Ölkühler in den Ölkreislauf gelangt.

Folgen: Dies kann zu erheblichen Motorschäden führen. Es wird dringend der Besuch einer Fachwerkstatt empfohlen.

| | |
|---|---|
| Reihe IV: | Kraftstoff im Öl (Verbrennungszustand durch Kraftstoffverdünnung) |
| | |
| Bereich G: | Es ist kein erhöhter Kraftstoffanteil im Öl nachzuweisen. |
| Bereich H: | Es befindet sich ein leicht bis mittel erhöhter Kraftstoffanteil im Öl. Es kann bereits zu einer Verdünnung des Öls und zu verschlechterten Schmiereigenschaften kommen. |
| Bereich I: | Es gelangt unverbrannter Kraftstoff an den Kolben vorbei ins Motoröl. |

Ursachen für Kraftstoff im Motoröl können eine mangelhafte Einspritzung, eine falsche Einstellung von Vergaser, Zündung oder der Ventilsteuerung sein. Auch die Einspritzdüsen oder die Kolbenringe können defekt sein. Die Folgen sind mangelhafte Schmiereigenschaften durch Verdünnung des Öls, eine Gefahr von Überhitzung, erhöhter Verschleiß, hoher Kraftstoffverbrauch oder schlechte Emissionswerte. Es wird dringend der Besuch einer Fachwerkstatt empfohlen.

Nach erfolgter Auswertung im externen Server 14 erfolgt eine Übermittlung der Testergebnisse an das Testaufnahmegerät oder an den am Testaufnahmegerät mittels USB-Verbindung 17 angeschlossenen Rechner 16, beispielsweise einen Laptop. Es erfolgt eine Anzeige und/oder eine Protokollierung der Eigenschaften des Motoröles und der jeweiligen zugeordneten Betriebszustände des Dieselmotores auf der Anzeigeeinheit 15 und/oder der Protokolliereinheit auf dem Testaufnahmegerät bzw. auf dem angeschlossenen Rechner 16.

Der Tester erhält eine gedruckte Analyse des "Gesundheitszustandes" seines Motors und kann die empfohlenen Maßnahmen einleiten.

### Bezugszeichenaufstellung

- 1: Kamera
- 2: Lichtquelle
- 3: Auflage (Trägerplatte)
- 4: Gehäuse
- 5: Rechner
- 6: Energieanschluss
- 7: Schnittstelle
- 8: Deckel
- 9: Scharnier
- 10: Testmedium
- 11: Arretierung
- 12: Aufnahmeschlitz
- 13: Scannereinheit
- 14: externer Server
- 15: Anzeigeeinheit
- 16: Rechner
- 17: USB-Verbindung
- 18: Funkverbindung

- A: gut
- B: mittel
- C: schlecht
- D: kein Wasser im Öl
- E: etwas Wasser im Öl
- F: viel Wasser im Öl
- G: kein Treibstoff im Öl
- H: etwas Treibstoff im Öl
- I: viel Treibstoff im Öl

## Patentansprüche

1. Verfahren zur Analyse von Ölen und technischen Betriebsflüssigkeiten und zur qualifizierten Bewertung der Betriebszustände von Aggregaten, bei welchem man einen Tropfen der zu untersuchenden Testflüssigkeit auf ein geeignetes Testmedium aufträgt, in das Testmedium eindringen lässt und nach einer vorgewählten Zeit derart auswertet, indem ein sich ergebendes Testbild optisch mit den Daten von mehreren Referenzbildern nach mehreren Testkriterien verglichen wird,
**gekennzeichnet durch** folgende Verfahrensschritte:
- Aufnahme und Speicherung der Daten mehrerer Reihen von Referenzbildern für verschiedene Testkriterien und Zuordnung der Referenzbilder zu bestimmten Eigenschaften der zu untersuchender Flüssigkeiten und Betriebszuständen von Aggregaten,
- Aufnahme und Speicherung des sich auf dem Testmedium (10) bildenden Testbildes des Tropfens mit einem Bildaufnahmegerät mindestens in einer Frontaufnahme und einer Rücklichtaufnahme,
- rechnergestützter Vergleich der Daten der Testbilder mit den Daten der Referenzbilder und Zuordnung der Daten eines oder mehrerer Bilder aus der Front- und der Rücklichtaufnahme zu den Referenzbildern für jeweils ein Testkriterium, welches die größte Übereinstimmung aufweist,
- Anzeige und/oder Protokollierung der Eigenschaften der Testflüssigkeit und/oder des Betriebszustandes des Aggregates, die den zugeordneten Daten der Referenzbilder entsprechen.

2. Verfahren nach Anspruch 1, **dadurch gekennzeichnet, dass** eine Aufnahme und Speicherung des sich auf dem Testmedium bildenden Testbildes des Tropfens mit dem Bildaufnahmegerät mittels einer UV-Rücklichtaufnahme erfolgt.

3. Verfahren nach Anspruch 1 oder 2, **dadurch gekennzeichnet, dass** der rechnergestützte Vergleich mittels einer Software erfolgt, die geeignet ist, die Form und/oder die Farbspektren der Daten der Testbilder und die Daten der Referenzbilder zu unterscheiden und die Daten der Bilder aus der Front- und der Rücklichtaufnahme zu den Referenzbildern für jeweils ein Testkriterium zuzuordnen.

4. Verfahren nach einem der Ansprüche 1 bis 3, **dadurch gekennzeichnet, dass** die Aufnahme und Speicherung der Daten der Referenzbilder auf einem externen Server (14) erfolgt und dass der rechnergestützte Vergleich auf dem externen Server (14) erfolgt, zu dem eine Online-Verbindung möglich ist.

5. Verfahren nach einem der Ansprüche 1 bis 4, **dadurch gekennzeichnet, dass** für die Frontaufnahme und/oder die Rücklichtaufnahme unterschiedlich farbige Lichtquellen (2) eingesetzt werden.

6. Verfahren nach einem der Ansprüche 1 bis 5, **dadurch gekennzeichnet, dass** durch das Bildaufnahmegerät eine Aufnahme zur Identifizierung und Dokumentation für eine Zuordnung der Testflüssigkeit zu einer Probennahme und/oder einem Objekt und/oder einer Person erstellt wird.

7. Verfahren nach einem der Ansprüche 1 bis 6, **dadurch gekennzeichnet, dass** ein Testmedium (10) verwendet wird, aufweisend ein Flächengewicht von 50,0 g/m² bis 200,0 g/m², umfassend, bezogen auf das Gesamtgewicht des Testmediums, 70,0 Gew.-% bis 98,0 Gew.-% Baumwollpulp, 0,0 Gew.-% bis 25,0 Gew.-% Cellulose und 0,5 Gew.-% bis 30,0 Gew.-% Kieselsäure und/oder mindestens eines Silikates.

8. Verfahren nach einem der Ansprüche 1 bis 7, **dadurch gekennzeichnet, dass** zur Analyse des Gesundheitszustands von Aggregaten Testflüssigkeit hinsichtlich enthaltener Verunreinigungen durch Ruß, Staub, Metallabrieb untersucht wird, indem ein in der Mitte des Testbildes in einem Bereich a entstehender Rußfleck mit den Daten der Referenzbilder verglichen und der Gehalt an Verunreinigungen ermittelt und bewertet wird.

9. Verfahren nach einem der Ansprüche 1 bis 8, **dadurch gekennzeichnet, dass** zur Analyse des Gesundheitszustands von Aggregaten Testflüssigkeit hinsichtlich des Zustandes der Testflüssigkeit untersucht wird, indem ein in einem Bereich b des Testbildes entstehender Kreisring bezüglich seiner Farbe mit den Daten der Referenzbilder verglichen und die Qualität der Testflüssigkeit in Relation zu seiner Alterung, beispielsweise der Öllaufleistung, ermittelt und bewertet wird.

10. Verfahren nach einem der Ansprüche 1 bis 9, **dadurch gekennzeichnet, dass** zur Analyse des Gesundheitszustands von Aggregaten Testflüssigkeit hinsichtlich des Gehaltes an Wasser und/oder im Wasser vorhandene Kühlflüssigkeit (z. B. Glykol) untersucht wird, indem eine in einem Bereich c des Testbildes entstehende gezackte Randzone bezüglich seiner Form mit den Daten der Referenzbilder verglichen und der Wassergehalt der Testflüssigkeit in Relation zum Zustand des Aggregates ermittelt und bewertet wird.

11. Verfahren nach einem der Ansprüche 1 bis 10, **dadurch gekennzeichnet, dass** zur Analyse des Gesundheitszustands von Aggregaten Testflüssigkeit hinsichtlich des Gehaltes an Kraftstoff untersucht wird, indem am äußeren Ring des Testbildes in einem Bereich d entstehender Treibstoffkreis bezüglich seiner Form und Farbe mit den Daten der Referenzbilder verglichen und der Treibstoffgehalt der Testflüssigkeit in Relation zum Zustand des Aggregates ermittelt und bewertet wird.

12. Einrichtung zur Analyse von Ölen und technischen Betriebsflüssigkeiten und zur qualifizierten Bewertung der Betriebszustände von Aggregaten zur Durchführung eines Verfahrens nach Anspruch 1, **gekennzeichnet durch**:
- eine Auflage (3) für ein flächenhaftes Testmedium (10), auf welches ein Tropfen der zu untersuchenden Testflüssigkeit auftragbar und ein Testbild erstellbar ist,
- mindestens ein Bildaufnahmegerät für eine Frontaufnahme und eine Rücklichtaufnahme,
- mindestens je eine Lichtquelle (2) zur Bilderfassung für eine Frontaufnahme und eine Rücklichtaufnahme,
- Mittel zur Speicherung der Daten mehrerer Reihen von Referenzbildern,
- Mittel zur Speicherung des sich auf dem Testmedium (10) bildenden Testbildes,
- Mittel, insbesondere eine Software, die geeignet ist, die Daten des Testbildes bezüglich seiner Form und Farbspektren mit den Daten der Referenzbilder zu vergleichen und die Daten je eines Bildes oder mehrerer Bilder aus der Front₋ und der Rücklichtaufnahme zu je einem Referenzbild oder Referenzbildern für jeweils ein Testkriterium zuzuordnen, welches die größte Übereinstimmung aufweist,
- eine Anzeigeeinheit (15) und/oder eine Protokolliereinheit zur Darstellung und/oder Ausgabe der Analyse- und/oder Bewertungsergebnisse.

13. Einrichtung nach Anspruch 12, **dadurch gekennzeichnet, dass** eine Lichtquelle (2) für eine UV-Rücklichtaufnahme vorhanden ist und das Bildaufnahmegerät geeignet ist, eine UV-Rücklichtaufnahme zu erfassen.

14. Einrichtung nach Anspruch 12 oder 13, **dadurch gekennzeichnet, dass** das Bildaufnahmegerät eine digitale Kamera (1) ist.

15. Einrichtung nach Anspruch 12 oder 13, **dadurch gekennzeichnet, dass** das Bildaufnahmegerät eine Scannereinheit (13) mit einem optisch-digitalen Scanner ist, beispielsweise ein Laserscanner.

16. Einrichtung nach einem der Ansprüche 12 bis 15, dadurch gekennzeichent, dass die Einrichtung eine Schnittstelle (7) zu einem Netzwerk und/oder zum Internet besitzt und sich die Mittel zur Speicherung der Daten mehrerer Reihen von Referenzbildern sowie die Software für den Vergleich und die Zuordnung der Daten der Testbilder mit den Daten der Referenzbilder auf einem externen Server befinden, zu dem eine Online-Verbindung möglich ist und die Einrichtung als Client-Server-Gerät betreibbar ist.

17. Einrichtung nach einem der Ansprüche 12 bis 16, dadurch gekennzeichent, dass das Testaufnahmegerät ein mobiles Endgerät mit einer eigenen Stromversorgung ist.

18. Einrichtung nach einem der Ansprüche 12 bis 17, **dadurch gekennzeichnet, dass** die Aufnahme für das flächenhafte Testmedium (10) eine Arretierung (11) zum Fixieren des Testmediums (10) besitzt.

## Claims

1. Method for the analysis of oils and technical service fluids and for the qualified evaluation of the operating states of units, wherein a drop of the test fluid to be examined is applied to a suitable test medium, is allowed to penetrate into the test medium and is assessed after a preselected time by a resulting test image being optically compared with the data of a plurality of reference images according to a plurality of test criteria,
**characterized by** the following method steps:
- recording and storage of the data of a plurality of series of reference images for different test criteria and assignment of the reference images to specific properties of the fluids to be examined and operating states of units,
- recording and storage of the test image of the drop that forms on the test medium (10) by means of an image recording apparatus at least in a front recording and a back light recording,
- computer-aided comparison of the data of the test images with the data of the reference images and assignment of the data of one or a plurality of images from the front and the back light recording to the reference images for a respective test criterion which has the greatest correspondence,
- display and/or logging of the properties of the test fluid and/or of the operating state of the unit which correspond to the assigned data of the reference images.

2. Method according to Claim 1, **characterized in that** a recording and storage of the test image of the drop that forms on the test medium is carried out by the image recording apparatus by means of a UV back light recording.

3. Method according to Claim 1 or 2, **characterized in that** the computer-aided comparison is carried out by means of software suitable for differentiating the shape and/or the color spectra of the data of the test images and the data of the reference images and for assigning the data of the images from the front and the back light recording to the reference images for a respective test criterion.

4. Method according to any of Claims 1 to 3, **characterized in that** the recording and storage of the data of the reference images is carried out on an external server (14), and **in that** the computer-aided comparison is carried out on the external server (14), to which an online connection is possible.

5. Method according to any of Claims 1 to 4, **characterized in that** differently colored light sources (2) are used for the front recording and/or the back light recording.

6. Method according to any of Claims 1 to 5, **characterized in that** the image recording apparatus creates a recording for identification and documentation for an assignment of the test fluid to a sampling and/or an object and/or a person.

7. Method according to any of Claims 1 to 6, **characterized in that** a test medium (10) is used, having a weight per unit area of 50.0 g/m² to 200.0 g/m², comprising, relative to the total weight of the test medium, 70.0% by weight to 98.0% by weight of cotton pulp, 0.0% by weight to 25.0% by weight of cellulose and 0.5% by weight to 30.0% by weight of silicic acid and/or at least one silicate.

8. Method according to any of Claims 1 to 7, **characterized in that** for the analysis of the state of health of units test fluid is examined with regard to contained contaminants resulting from soot, dust, metal abrasion by a procedure in which a soot spot that arises in the center of the test image in a region a is compared with the data of the reference images and the content of contaminants is determined and evaluated.

9. Method according to any of Claims 1 to 8, **characterized in that** for the analysis of the state of health of units test fluid is examined with regard to the state of the test fluid by a procedure in which an annulus that arises in a region b of the test image is compared, with regards to its color, with the data of the reference images and the quality of the test fluid in relation to its aging, for example the oil running performance, is determined and evaluated.

10. Method according to any of Claims 1 to 9, **characterized in that** for the analysis of the state of health of units test fluid is examined with regard to the content of water and/or cooling fluid (e.g. glycol) present in the water by a procedure in which a jagged edge zone that arises in a region c of the test image is compared, with regard to its shape, with the data of the reference images and the water content of the test fluid in relation to the state of the unit is determined and evaluated.

11. Method according to any of Claims 1 to 10, **characterized in that** for the analysis of the state of health of units test fluid is examined with regard to the content of fuel by a procedure in which a fuel circle that arises at the outer ring of the test image in a region d is compared, with regard to its shape and color, with the data of the reference images and the fuel content of the test fluid in relation to the state of the unit is determined and evaluated.

12. Device for the analysis of oils and technical service fluids and for the qualified evaluation of the operating states of units for carrying out a method according to Claim 1, **characterized by**:
- a support (3) for a planar test medium (10), to which a drop of the test fluid to be examined can be applied and a test image can be created,
- at least one image recording apparatus for a front recording and a back light recording,
- at least in each case one light source (2) for image acquisition for a front recording and a back light recording,
- means for storing the data of a plurality of series of reference images,
- means for storing the test image that forms on the test medium (10),
- means, in particular software, suitable for comparing the data of the test image with regard to its shape and color spectra with the data of the reference images and for assigning the data of in each case one image or a plurality of images from the front and the back light recording to in each case one reference image or reference images for a respective test criterion which has the greatest correspondence,
- a display unit (15) and/or a logging unit for representing and/or outputting the analysis and/or evaluation results.

13. Device according to Claim 12, **characterized in that** a light source (2) for a UV back light recording is present and the image recording apparatus is suitable for acquiring a UV back light recording.

14. Device according to Claim 12 or 13, **characterized in that** the image recording apparatus is a digital camera (1).

15. Device according to Claim 12 or 13, **characterized in that** the image recording apparatus is a scanner unit (13) having an optical-digital scanner, for example a laser scanner.

16. Device according to any of Claims 12 to 15, **characterized in that** the device has an interface (7) to a network and/or to the internet and the means for storing the data of a plurality of series of reference images and also the software for the comparison and the assignment of the data of the test images with the data of the reference images are situated on an external server to which an online connection is possible and the device can be operated as a client server apparatus.

17. Device according to any of Claims 12 to 16, **characterized in that** the test recording apparatus is a mobile terminal having a dedicated power supply.

18. Device according to any of Claims 12 to 17, **characterized in that** the receptacle for the planar test medium (10) has a locking element (11) for fixing the test medium (10).

## Revendications

1. Procédé d'analyse d'huiles et de fluides techniques et d'évaluation qualifiée des états de fonctionnement de groupes, dans lequel on dépose une goutte du fluide à analyser sur un support de test approprié, on la laisse pénétrer dans le support de test et, au bout d'une durée présélectionnée, on l'évalue en comparant optiquement l'image de test ainsi obtenue avec les données de plusieurs images de référence selon plusieurs critères de test,
**caractérisé par** les étapes consistant à :
- acquérir et mémoriser les données de plusieurs séries d'images de référence pour des critères de test différents et associer les images de référence à des propriétés déterminées des liquides à examiner et à des états de fonctionnement des groupes,
- acquérir et mémoriser l'image de test de la goutte se formant sur le support de test (10) au moyen d'un appareil d'acquisition d'images dans une acquisition de face et une acquisition de dos,
- comparer à l'aide d'un ordinateur les données des images de test avec les données des images de référence et associer aux images de référence les données d'une ou plusieurs images des acquisitions de face et de dos pour chaque critère de test, présentant la concordance la plus grande,
- afficher et/ou imprimer les propriétés du liquide de test et/ou l'état de fonctionnement du groupe, qui correspondent aux données associées des images de référence.

2. Procédé selon la revendication 1, **caractérisé en ce qu'**une acquisition et une mémorisation de l'image de test de la goutte se formant sur le support de test à l'aide de l'appareil d'acquisition d'images est effectuée au moyen d'une acquisition par rétroéclairage UV.

3. Procédé selon la revendication 1 ou 2, **caractérisé en ce que** la comparaison à l'aide d'un ordinateur est effectuée au moyen d'un logiciel qui est conçu pour différencier la forme et/ou les spectres de couleurs des données des images de test et les données des images de référence et pour associer les données des images des acquisitions de face et de dos aux images de référence pour un critère de test respectif.

4. Procédé selon l'une quelconque des revendications 1 à 3, **caractérisé en ce que** l'acquisition et la mémorisation des données des images de référence est effectuée sur un serveur externe (14) et **en ce que** la comparaison à l'aide d'un ordinateur est effectuée sur le serveur externe (14) auquel il est possible d'établir une connexion en ligne.

5. Procédé selon l'une quelconque des revendications 1 à 4, **caractérisé en ce que**, pour l'acquisition de face et/ou l'acquisition de dos, des sources lumineuses de couleurs différentes (2) sont utilisées.

6. Procédé selon l'une quelconque des revendications 1 à 5, **caractérisé en ce qu'**une acquisition permettant d'identifier et de documenter une association du liquide de test avec un prélèvement d'échantillons et/ou avec un objet et/ou avec une personne est établie.

7. Procédé selon l'une quelconque des revendications 1 à 6, **caractérisé en ce qu'**on utilise un support de test (10) ayant un poids par unité de surface de 50,0 g/m² à 200,0 g/m², comprenant, par rapport au poids total du support de test, 70,0 % en pds à 98,0 % en pds de pulpe de coton, 0,0 % en pds à 25,0 % en pds de cellulose et 0,5 % en pds à 30,0 % en pds d'acide silicique et/ou un silicate.

8. Procédé selon l'une quelconque des revendications 1 à 7, **caractérisé en ce que**, pour analyser l'état de santé des groupes, un liquide de test est examiné en ce qui concerne des impuretés provenant de la suie, de la poussière, de l'érosion du métal, en comparant une tache de suie apparaissant au milieu de l'image de test dans une zone a, aux données des images de référence et en déterminant et évaluant la teneur en impuretés.

9. Procédé selon l'une quelconque des revendications 1 à 8, **caractérisé en ce que**, pour analyser l'état de santé des groupes, un liquide de test est examiné en ce qui concerne l'état du liquide de test en comparant une couronne apparaissant dans une zone b de l'image de test du point de vue de sa couleur, aux données des images de référence et en déterminant et évaluant la qualité du liquide de test en relation avec son vieillissement, par exemple les performances kilométriques fournies par l'huile.

10. Procédé selon l'une quelconque des revendications 1 à 9, **caractérisé en ce que**, pour analyser l'état de santé des groupes, un liquide de test est examiné en ce qui concerne la teneur en eau et/ou en fluide réfrigérant présent dans l'eau (par exemple du glycol), en comparant une zone de bord dentelée apparaissant dans une zone c de l'image de test en ce qui concerne sa forme, aux données des images de référence et en déterminant et évaluant la teneur en eau du liquide de test en relation avec l'état du groupe.

11. Procédé selon l'une quelconque des revendications 1 à 10, **caractérisé en ce que**, pour analyser l'état de santé des groupes, un liquide de test est examiné en ce qui concerne la teneur en carburant en comparant un cercle de carburant apparaissant au niveau de l'anneau externe de l'image de test dans une zone d en ce qui concerne sa forme et sa couleur, aux données des images de référence et en déterminant et évaluant la teneur en carburant du liquide de test en relation avec l'état du groupe.

12. Dispositif d'analyse d'huiles et de fluides techniques et d'évaluation qualifiée de l'état de fonctionnement de groupes pour mettre en oeuvre un procédé selon la revendication 1, **caractérisé par** :
- une installation (3) destinée à un support de test étendu (10) sur lequel peut être déposée une goutte du liquide de test à examiner et une image de test peut être formée,
- au moins un appareil d'acquisition d'images pour une acquisition de face et une acquisition de dos,
- au moins une source lumineuse respective (2) destinée à détecter des images pour une acquisition de face et une acquisition de dos,
- des moyens destinés à mémoriser les données de plusieurs séries d'images de référence,
- des moyens destinés à mémoriser des images de test se formant sur le support de test (10),
- des moyens, notamment des logiciels, qui sont conçus pour comparer les données de l'image de test en ce qui concerne sa forme et ses spectres de couleurs aux données des images de référence et pour associer les données d'une image respective ou de plusieurs images de l'acquisition de face et de l'acquisition de dos à une image de référence respective ou à des images de référence pour un critère de test respectif, qui présente la concordance la plus grande,
- une unité d'affichage (15) et/ou une unité d'impression permettant de représenter et/ou de fournir en sortie les résultats de l'analyse et/ou de l'évaluation.

13. Dispositif selon la revendication 12, **caractérisé en ce qu'**il est prévu une source lumineuse (2) destinée à une acquisition par rétroéclairage UV et **en ce que** l'appareil d'acquisition d'images est approprié pour obtenir une acquisition UV de dos.

14. Dispositif selon la revendication 12 ou 13, **caractérisé en ce que** l'appareil d'acquisition d'images est une caméra numérique (1).

15. Dispositif selon la revendication 12 ou 13, **caractérisé en ce que** l'appareil d'acquisition d'images est une unité à scanner (13) comportant un scanner optique-numérique, par exemple un scanner à laser.

16. Dispositif selon l'une quelconque des revendications 12 à 15, **caractérisé en ce que** le dispositif possède une interface (7) avec un réseau et/ou avec l'Internet et **en ce que** les moyens de mémorisation des données de plusieurs séries d'images de référence et du logiciel destiné à comparer et à associer les données des images de test avec les données des images de référence se trouvent sur un serveur externe avec lequel une connexion en ligne est possible et **en ce que** le dispositif peut être mis en fonctionnement en tant qu'appareil de type client-serveur.

17. Dispositif selon l'une quelconque des revendications 12 à 16, **caractérisé en ce que** l'appareil d'acquisition d'images est un terminal mobile comportant sa propre alimentation électrique.

18. Dispositif selon l'une quelconque des revendications 12 à 17, **caractérisé en ce que** l'acquisition destinée au support de test étendu (10) possède un dispositif de blocage (11) destiné à fixer le support de test (10).
